# EUROPEAN PATENT APPLICATION

(11) **EP 1 375 646 A1**
(43) Date of publication of application: **02.01.2004**
(21) Application number: 02013838.4
(22) Date of filing: 21.06.2002
(51) Int. Cl.: C12N 5/08, C12N 5/10, C12N 15/18

(54) **Genetically engineered keratinocytes expressing at least one growth factor and/or activated EGF receptor, skin equivalent comprising said keratinocytes and toxicity assay using said keratinocytes**

(71) Applicant: Deutsches Krebsforschungszentrum Stiftung des öffentlichen Rechts, 69120 Heidelberg (DE)
(72) Inventor: Fusenig, Norbert, 69118 Heidelberg (DE); Maas-Szabowski, Nicole, 69118 Heidelberg (DE); Stärker, Anja, 69181 Leimen (DE); Stark, Hans-Jürgen, 74909 Meckesheim (DE); Martin, Iris, 68723 Plankstadt (DE); Goedecke, Eva, 74858 Aglasterhausen (DE)
(74) Representative: Schüssler, Andrea, Dr.

(57) **Abstract**

Described is an immortalized mammalian skin keratinocyte line, preferably HaCaT, stably transfected with a vector capable of expressing a nucleic acid sequence encoding at least one growth factor and/or activated EGF receptor. Furthermore, a skin equivalent and an in vitro toxicity assay making use of this immortalized mammalian skin keratinocyte line is described.

## Description

The present invention relates to an immortalized mammalian skin keratinocyte line, preferably HaCaT, stably transfected with a vector capable of expressing a nucleic acid sequence encoding at least one growth factor and/or activated EGF receptor. The present invention also relates to a skin equivalent and an in vitro toxicity assay making use of this transfected immortalized mammalian skin keratinocyte line.

In order to reduce cost-intensive animal experiments and to increase the number of samples to be tested at the same time, cell cultures are increasingly used as in vitro test systems in the pharmacologic and cosmetic industry and research. As far as skin testing is concerned, monolayers of freshly isolated keratinocytes from human skin but also established cell lines are utilized to assess organ-specific reactions. Such monolayer cultures of keratinocytes, however, reveal only limited aspects of the differentiated epidermis of the skin. In such keratinocyte monolayer cultures, the ordered stratification with step-wise differentiation from the basal layer to the stratum corneum representing the typical barrier function of the epidermis is not formed. To correct this deficit, organotypic cocultures consisting of keratinocytes growing on different matrices, mostly based on collagen, with incorporated skin fibroblasts have been developed as in vitro skin equivalents. Those cultures exhibit a normal differentiating squamous epithelium with development of a stratum corneum comparable to the epidermis of the skin. Such cocultures established from foreskin keratinocytes and fibroblasts with collagen type I gel have been commercially available for a short period and used for in vitro toxicity tests (Organogenesis, Boston, USA). Due to biologic, but also methodologically caused variations in the quality of such cultures but also due to cost reasons, the utilization of these complex cultures was limited so that the marketing has been discontinued.

Different keratinocyte cell lines have been developed, mostly by transfection with viral oncogenes (e.g. SV40, HPV 16) and used in organotypic cultures as unlimited available substitutes of normal keratinocytes. However, the epithelia produced by these cell lines exhibited irregular structure and aberrant or lacking differentiation. A spontaneously immortalized cell line (HaCaT) which, in contrary to the transfected cell lines, has maintained in culture as well as in transplants on nude mice a remarkably good differentiation capacity has been established and characterized. In organotypic cocultures, these cells form a multilayered epithelium, however with delayed kinetics and incomplete differentiation. In order to improve the structural organization, proliferation and differentiation of HaCaT keratinocytes in organotypic cultures, the number of skin fibroblasts embedded in the collagen matrix had been increased or a cell-free dermal skin matrix (deepidermized dermis, DED) had been used. Both methods lead to an improved stratification and differentiation of the HaCaT epithelium, however with a considerable delay in culture time and with some deficiencies as compared to normal skin keratinocyte cultures. By modification of the culture medium and transfection of the HaCaT cells of the anti-apoptotic gene bcl-2 or the ras oncogene, respectively, proliferation of these cells could be stimulated even when growing on a collagen gel but the deficient differentiaton of a stratified epithelium could not be significantly improved. Thus, the HaCaT skin equivalents produced with the methods known so far exhibit distinct deficiencies in their differentiation capacity as compared to normal skin keratinocytes both as far as functional organization and keratinization are concerned. The stratum corneum characteristic for normal epidermis and essential for the barrier function is either completely missing or was only slightly indicated and incompletely formed (e.g. parakeratotic, i.e. with remnant nuclei). Moreover, the epithelium formed by HaCaT keratinocytes showed marked deficiencies in the synthesis of the essential skin lipids, components which are important players in the barrier function of the epidermis. Moreover, these HaCaT skin equivalents exhibited major variations concerning epithelial structure and differentiation, probably due to the difficulties to standardise influences of matrix and fibroblasts and to deficiencies in signal reception and transduction in keratinocytes.

Thus, the technical problem underlying the present invention is to provide an immortalized mammalian skin keratinocyte line which does not exhibit the above disadvantages and which is suitable for establishing an in vitro skin equivalent which allows a sensitive, reliable, cost saving and quantitative routine analysis of any skin affecting, e.g., damaging factor.

The solution to the above technical problem has been achieved by providing the embodiments characterized in the claims.

During the experiments leading to the present invention it was found that the deficiency of HaCaT mentioned above was caused by the lacking or strongly reduced expression of essential cytokines (e.g. 11-1) and growth factors (e.g. TGFα) in the HaCaT cells and the delayed expression of receptors for growth factors, such as KGF and GM-CSF, which are induced in normal keratinocyte cocultures, namely via Il-1 in the cocultured fibroblasts. By stimulation of the HaCaT cells in organotypic cultures by growth factors (preferably TGFα or EGF; both bind to the same receptor) or activated EGF receptors, the expression of the receptors for KGF and GM-CSF is markedly elevated. Thereafter the addition of KGF and GM-CSF or of its inducer Il-1 lead to an increased proliferation and differentiation of HaCaT cells in organotypic cocultures.

The deficiency of the HaCaT cells concerning the inducibility of their own growth factors in the cocultivated fibroblasts as well as their reduced response to these factors was repaired by the stable transfection and expression of at least one growth factor and/or activated EGF receptor. Preferably, the growth factor is a EGF receptor binding growth factor, in particular the autocrine acting growth factor TGFα (transforming growth factor α), EGF, HB-EGF or amphiregulin or parts thereof which are necessary for signal transduction. The activated EGF receptor is preferably activated by introducing mutations into the wild type form.

To achieve this, in one preferred embodiment of the present invention, TGFα encoding nucleic acid sequences were transfected under a constitutive active promoter (CMV). Stably transfected HaCaT cells with optimal production of this growth factor were expanded and used in organotypic cocultures. These HaCaT transfectants, constitutively expressing TGFα, are now capable, in cocultures with fibroblasts, to reconstitute a rather normal skin epithelium with all differentiation functions. By use of defined numbers of resting (postmitotic) fibroblasts within the collagen gel and addition of further growth and differentiation factors (e.g. GM-CSF, TGFβ) a standardized and reproducible method to produce differentiating skin equivalents with immortal HaCaT cells has been achieved. These in vitro skin equivalents are well suited for the routine testing of biocompatibility as well as efficacy of skin active agents, as used in cosmetics and dermatology. A further advantage is that the standardized and reproducible in vitro skin equivalents can be produced with immortal human keratinocytes (available in unlimited amounts) and within a one-to-two-week culture period. These skin equivalents exhibit all essential functions of organotypic cocultures of normal keratinocytes and are very much comparable to normal epidermis in the skin. Using immortalized human or murine fibroblast cell lines (e.g. Wi26 or 3T3, respectively) these in vitro skin equivalents can be generated without any primary cultures, i.e. without new cell isolation from skin specimens. Thus, biologically and methodologically caused variations in the production and reproducibility of such organotypic cultures as well as a major cost factor produced the primary cultures have been successfully eliminated.

To summarize, a novel mammalian skin equivalent has been developed allowing to establish a highly sensitive and quantitative in vitro assay for identifying and characterizing environmental hazards or agents affecting skin. This assay utilizes in vitro skin equivalents with growth factor(e.g. TGFα)-transfected immortal (e.g. HaCaT)-keratinozytes. This skin equivalent is fully functioning and useful for various in vitro studies and test assays. The skin equivalent exhibits comparable functional competence (as regards cell growth, stratification and structural organisation, differentiation and keratinization as well as barrier function of the epidermis) as compared to in vitro equivalents made of normal keratinocytes.

Thus, in one aspect, the present invention relates to an immortalized mammalian keratinocyte line stably transfected with a vector capable of expressing a nucleic acid sequence encoding at least one growth factor and/or activated EGF receptor. Preferably the growth factor is TGFα and/or EGF, more preferably human TGFα and EGF, respectively.

By "mammalian", it is meant that the keratinocyte line can be established from any mammalian keratinocyte. Humans are the preferred mammal. However, the invention can be practiced with other mammals such as non-human primates and members of the bovine, ovine, porcine, equinine, canine and feline species as well as rodents such as mice, rats and guinea pigs and members of the lagomorph family including rabbit.

As used herein, the term "immortalized mammalian keratinocyte line" relates to any immortalized mammalian keratinocyte line which can be transfected with vectors, allows gene expression and is useful in the assay of the invention. Examples of suitable human cell lines are skin and mucosal keratinocytes immortalized by transfection of the viral oncogenes of Simian virus 40 (SV40-LT gene), of human papilloma viruses type 16, 18, 33 (HPV-E6/E7 gene) or by transfection of the catalytical subunit of the enzyme telomerase. In a preferred embodiment, this cell line is the cell line HaCaT or a cell line having the functional characteristics of HaCaT.

The construction of the vectors needed for obtaining a genetically engineered immortalized mammalian keratinocyte line of the invention can be carried out according to conventional methods (cf. Sambrook et al., 1989, Molecular Cloning: A Laboratory Manual, 2^{nd} edition, Cold Spring Harbor Laboratory Press, NY, USA) . Suitable starting vectors allowing amplification in prokaryotic and eukaryotic expression systems including the immortalized mammalian keratinocyte lines described above are, e.g. the "pGL-3" basic-vector (Promega GmbH, Mannheim, Germany) or any other shuttle vector allowing amplification in procaryotes and expression in eucaryotes. These vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts for amplifying said vectors include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells; and plant cells.

Introduction of the above described vectors into the host cell can be effected by well known methods, e.g. calcium phosphate transfection, DEAE-dextran mediated transfection, cationic lipid-mediated transfection, electroporation, transduction, infection, or other methods. Such methods are described in many standard laboratory manuals.

As used herein, the terms "TGFα", "EGF", "HB-EGF" and "amphiregulin" relate to biologically active growth factors having an amino acid sequence as described in the literature or an amino acid sequence differing from such amino acid sequence, e.g., due to substitutions, deletions or insertion of amino acid residue(s) which, however, represent biologically active growth factors.

In a preferred embodiment of the invention a double or multiple transfection with the above mentioned growth factors and/or additionally KGF, GM-GSF, FGF-10, FGF-22 and EL-20 is carried out after successful transfection of the first factor or receptor.

The nucleic acid sequence encoding the growth factor, preferably TGFα or EGF (Gray et al., Nature 303 (1983), pp. 722-725), and/or activated EGF receptor is under the control of any promoter which is functional in mammalian, preferably mammalian cells with constitutive and strong promoters, e.g. viral promoters, being preferred. A particularly preferred promoter is the CMV promoter.

The most preferred embodiment of the immortalized mammalian skin keratinocyte line of the present invention is HaCaT containing the vector pIRES neo2-TGFα ("Cell Culture HaCaT-TGFalpha-T10" deposited under the Budapest Treaty with the "Deutsche Sammlung von Mikroorganismen und Zellkulturen GmbH (DSMZ), Mascheroder Weg 1b, Braunschweig, Germany" on June 5, 2002: DSM ACC2545

The present invention also relates to an immortalized mammalian skin keratinocyte line as described above further containing a reporter gene operatively linked to a promoter inducible by environmental hazards. Preferably, this reporter gene is inserted into a vector which is capable of stably transfecting said cell line. This vector can be the vector already expressing the growth factor encoding gene or can be a separate vector.

As used herein, the term "a promoter inducible by environmental hazards" relates to any promoter which is inducible by factors which show cell toxicity, e.g. can lead to a damage of cells. Such promoters and suitable sources for such promoters are well known to the person skilled in the art. The expression vectors (inducible promoter/reporter gene) will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated. Particular promoters are selected according to the nature of the stress factor to be analysed. As promoter constructs to serve as sensitive indicators for a broad spectrum of environmental hazards from UVR over chemicals to pesticides and industrial waste products, those are selected which promise both broad sensitivity and high specificity to certain kinds of agents. The promoters can be selected according to the known biologic mechanisms of skin irritation, i.e. consisting of both direct alterations induced primarily in the first-line target cells, the keratinocytes, and indirect effects caused in fibroblasts and endothelial cells by mediators (e.g. cytokines, prostaglandins) released by injured keratinocytes. For example, the promoter of HSP-70, the heat shock protein 70, can be used to serve as a broad-range stress indicator, sensitive to activation by many agents including heat, UVR, reactive oxygen species (ROS), metals, and chemicals. HSP-27 is another cellular stress protein activated by a spectrum of events including disturbances of cell differentiation and is also induced by teratogenic as well as hormone-like agents. Thus, the HSP-27 promoter will serve as a sensitive indicator for endocrine disrupters (e.g. xenoestrogens). NF_{K}B is a transcription factor activated by various stimuli and will, thus, serve as indicator both for direct (UVR) as well as indirectly (ROS) caused cell irritation. The transcription factor AP-1, has a similar, although different activation spectrum. Thus, NF_{K}B or AP-1 sites containing promoters can be used for assaying the above stress factors. Examples of environmental hazards are heat, ultraviolet irradiation (UVR), ozone or an organic or anorganic compound, e.g. a metal, pesticide, herbicide, biocide or a combustion product.

As used herein, the term "reporter gene" relates to any gene the product of which can be used in order to determine the rate of expression directed by the inducible promoter. Such reporter genes and assays for determining the expression rate of the reporter gene as well as for appropriately linking the reporter gene to the promoter are well known to the person skilled in the art. One example of such a reporter gene is the gene encoding the human placental secreted alkaline phosphatase (SEAP). The SEAP reporter gene encodes a truncated form of the placental alkaline phosphatase enzyme which lacks the membrane anchoring domain, thereby allowing the protein to be efficiently secreted by transfected cells. Levels of SEAP activity detected in the culture medium are directly proportional to changes in intracellular concentrations of SEAP mRNA and protein. SEAP is extremely heat-stable and resistant to the inhibitor L-homoarginine, so that endogenous alkaline phosphatase activity can be eliminated by pretreatment of samples at 65°C and incubation with this inhibitor. A commercial detection kit composed of chemiluminescent substrate (CSPD) and the fluorescent substrate 4-methylumbelliferyl phosphate (MUP) enables to monitor expression of the SEAP reporter gene through simple, sensitive, non-radioactive assays (e.g. provided by Tropix/Becton-Dickinson, Heidelberg, Germany). The chemiluminescent assay can detect as little as 10⁻¹³ g of SEAP protein, making it one of the most sensitive enzymatic reporters available comparable to assays for firefly luciferase. The detection assay is linear over a 10⁴-fold range of enzyme concentrations, which makes it particularly well-suited for dose-response studies and comparative analyses. The secreted nature of SEAP provides several advantages for the use of this enzyme as a transcription reporter, because i) preparation of cell lysates is not required, ii) the kinetics of gene expression can be studied simply be repeated collection of the culture medium from the same cultures, iii) toxicity assays after both single and multiple exposure of cells to damaging agents can be performed simultaneously, iv) sample collection of the culture medium can be automated in 96-well plates, and iv) background from endogenous alkaline phosphatase is almost absent in the culture medium following pretreatment. Additional well known reporter molecules that can be used instead of SEAP, which are, however, not secreted into the culture medium include chloramphenicol acetyltransferase (CAT), luciferase, alkaline phosphatase, β-galactosidase, green fluorescent protein (GFP) or any protein detectable by an chemiluminescent assay or an antibody.

The present invention also relates to a mammalian skin equivalent comprising a discrete epidermal layer of the immortalized mammalian skin keratinocyte line of the present invention on top of a gel matrix comprising collagen or any matrix produced by skin fibroblasts cultured in 3D-scaffolds, preferably a collagen matrix, containing fibroblasts (either proliferating (2-4 x 10⁵/ml) or postmitotic cells (2-6 x 10⁵/ml)). The person skilled in the art can establish an mammalian skin equivalent according to the present invention by applying the method described in Example 1, below, or well known methods, e.g. methods described in Boukamp et al., Cancer Res. 45:5582-5592 (1985) or in Fusenig et al., Bull. Cancer, 65:271-280 (1978), Fusenig, et al., J. Invest. Dermotol. 81:168s-175s (1983) (comprising preparing collagen gels with fibroblasts in a silicon chamber and later seeding the gel with keratinocytes) or EP-A1 1 130 086. This mammalian skin equivalent can be formed, maintained and used essentially in tissue culture thereby circumventing the need for using animals to test products which may be harmful to the skin. For example, companies which are in the practice of testing products on animals to determine if the products cause skin discoloration, aggravation, etc., can test the products on the mammalian skin equivalent provided herein wherein the skin equivalent is formed and maintained in tissue culture. The particular mammalian skin equivalent which will be formed will be dependent on the source of the keratinocytes and fibroblasts, e.g., when human keratinocytes and fibroblasts are used to form the skin equivalent, a human skin equivalent is formed.

Moreover, the present invention also relates to a mammalian skin equivalent comprising a discrete epidermal layer composed of (a) the immortalized mammalian skin keratinocyte line of the present invention and (b) keratinocyte cells permanently transfected with a reporter gene operatively linked to a promoter inducible by environmental hazards. Such particular type of mammalian skin equivalent has an epidermis of increased sensitivity and indicator functions for testing in cosmetics and pharmaco-toxicology.

The present invention also relates to a method for forming a mammalian skin equivalent of the present invention comprising
(a) preparing a gel matrix, preferably a collagen matrix, with or without embedded fibroblasts and applying a suitable medium on the surface of said matrix;
(b) replacing the medium with a suspension comprising cells of the immortalized mammalian skin keratinocyte line of the present invention; and
(c) cultivating the cells of (a) and (b).

In a preferred embodiment the fibroblasts may be primary, proliferating or postmitotic fibroblasts or in form of a fibroblast cell line.

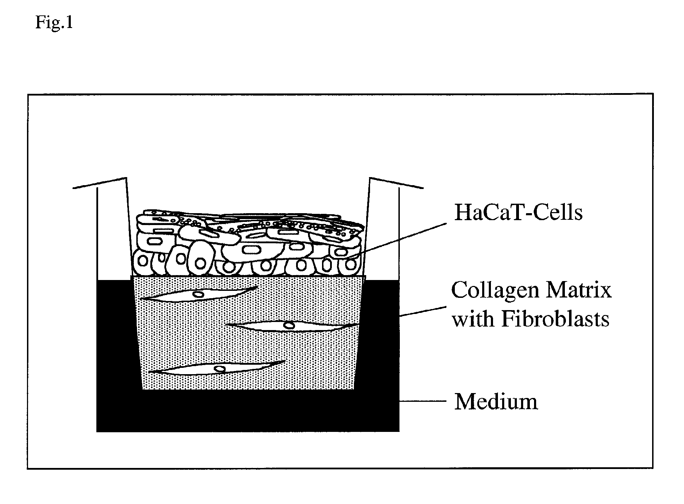

In a more preferred embodiment, the method of the present invention further comprises the addition of cytokines with function on skin growth and differentiation, preferably (a) Il-1 or (b) KGF and/or GM-CSF, during cultivation of the cells, preferably to the medium of the lower culture compartment; see, e.g., Example 1(H) and Figure 1. The concentration of said agents, preferably, is in the range of 1 to 10 ng/ml medium (in the case of Il-1/KGF) or 10-100 ng/ml (in the case of GM-CSF).

In a preferred embodiment, the method uses a suspension further comprising keratinocyte cells permanently transfected with a reporter gene operatively linked to promoter inducible by environmental hazards.

In the most preferred embodiment of the method of the present invention the fibroblasts are resting postmitotic fibroblasts which can be prepared according to well known methods, e.g. the method detailed in Example 1 (F) or using mitomycin c (Rheinwald et al., Cell 6 (1975), pp. 331-335).

Finally, the present invention relates to an in vitro assay for identifying a candidate agent or environmental hazard having an affect on skin comprising
(a) providing the mammalian skin equivalent of the present invention;
(b) contacting said skin equivalent with a candidate agent or applying a candidate environmental hazard to said skin equivalent; and
(c) determining whether said candidate agent or environmental hazard has an effect on said skin equivalent or whether expression of the reporter gene occurs, wherein expression of the reporter gene is indicative of cell toxicity of the candidate environmental hazard.

This in vitro assay can also be carried out by (a) culturing the immortalized mammalian skin keratinocyte line of the present invention in (i) the presence and (ii) absence of the candidate agent or candidate environmental hazard. This assay, i.e. the culturing of the cell, contacting the cell with the candidate agent or candidate environmental hazard and the determination of the concentration of the protein expressed from the reporter gene can be carried according to well known routine methods and assays, e.g. the methods and assays described in the examples, below.

In the assay of the present invention, the candidate agent can be any agent which may modify the phenotype, genotype or formation of the skin equivalent. The candidate agent can be in the form of a gene administered to the keratinocytes and/or fibroblasts, or in the form of the gene product. Candidate agents include any cosmetic, therapeutic or pharmaceutical including anti-cancer and anti-aging agents including genes, oligonucleotides, peptides, radiation, and any other toxic or non-toxic agent. Candidate agents also include those components normally expressed by skin cells as described herein, or inhibitors of those components. Identification of candidate agents which have an affect on the phenotype, genotype or formation of mammalian skin equivalents are useful for diagnostic and therapeutic purposes in dermatology. In particular, candidate agents which are identified as having beneficial effects can be formed into cosmetic or pharmaceutical formulations and then administered. Alternatively, candidate agents identified as effective agents can be used to treat the cells ex vivo and then the treated cells can be administered to the individual in the form of a slurry or an already formed skin equivalent. Similarly, agents which are identified as harmful to the phenotype, genotype or formation of mammalian skin equivalents of the present invention are also useful for diagnostic and therapeutic purposes in dermatology. In particular, they are useful to identify cures and treatments to prevent or reverse the affect of such an agent. Finally, agents which are identified as having no affect to the phenotype, genotype or formation of skin equivalents are useful in formulations for cosmetics and pharmaceuticals which will be safe for use on skin.

### Brief description of the drawings

### Figure 1: Schematic illustration of a HaCaT organotypic coculture with HaCaT epithelial cells growing air-exposed on top of a fibroblast-containing collagen gel forming a stratified epidermis

Cells are nourished by diffusion of the culture medium through the collagen gel (from below).

### Figure 2: Morphology (H&E stainings of histological sections) of HaCaT organotypic cocultures

HaCaT cells were grown for 14 days as organotypic cocultures on collagen gels containing 2x10⁵/ml resting fibroblasts in DMEM with 10% FCS and ascorbic acid. As additives the culture medium contained additionally KGF; GM-CSF, 11-1, EGF or TGFα and EGF + TGFα. For comparison, a 10-day-old organotypic coculture of normal human keratinocytes (NEK) on fibroblasts-containing collagen gel is shown. (Figures are of the same magnification, bar indicates 100µm).

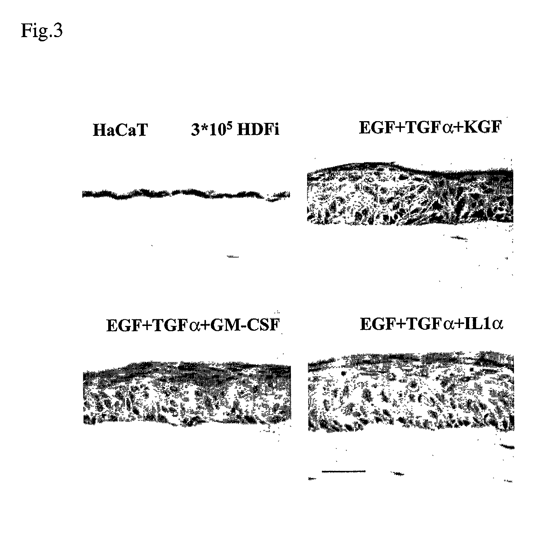

### Fig. 3: Histologic cross sections (H&E stainings of organotypic cocultures) on collagen with 2x10⁵ human fibroblasts/ml in the collagen gel grown in DMEM + 10% FCS and ascorbic acid

The medium was supplemented by EGF and TGFα and contained in addition either KGF, GM-CSF or Il-1α. The increased differentiation visible at the enlarged stratum corneum and the appearance of a stratum granulosum is obvious in organotypic cultures after addition of GM-CSF and Il-1α, respectively. (Same magnification in all figures, bar represents 100µm).

### Figure 4

(a) Schematic representation of the vector used for stable transfection
   The cDNA of TGFα has been cloned, starting at position 1 until position 560 in the EcoRI and BglI restriction sites of the multiple cloning site in the pIRESneo2 (Clonetech) expression vector. In this vector the expression of the human TGFα cDNA is controlled by the CMV promoter.
(b) Sequence of the TGFα EcoRI/pBR 327 vector (ATCC no. 59950) as well as the pIRESneo2-TGFα vector.

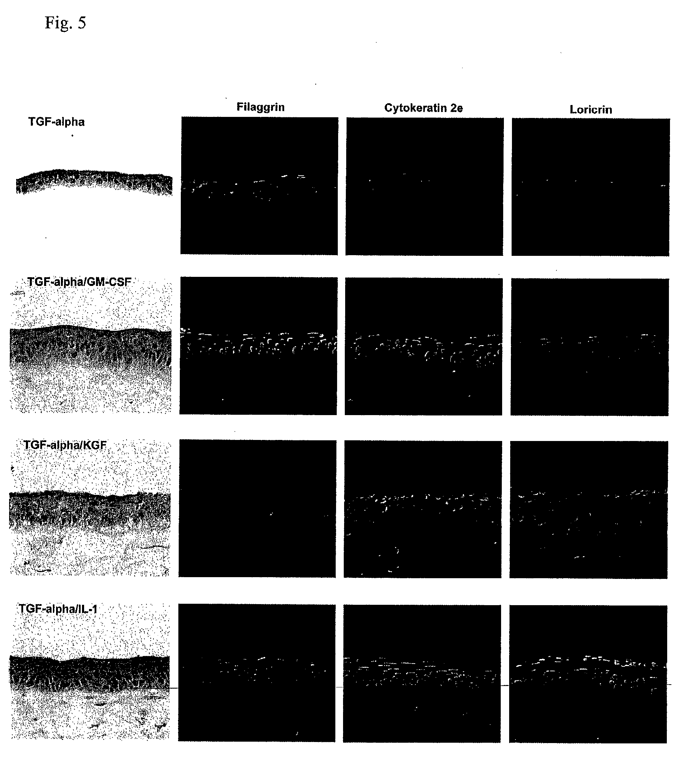

### Figure 5: Differentiation markers in HaCaT organotypic cocultures grown for two weeks with 2x10⁵ resting fibroblasts in a collagen gel and cultured in DMEM with 10% FCS + ascorbic acid and TGFα (first row), following addition of GM-CSF (second row), KGF (third row), or Il-1α (fourth row)

Fixed sections were stained by hematoxilin-eosin (first vertical row). Frozen sections were cut from organotypic cultures and the differentiation-associated proteins filaggrin (in red, second vertical row), keratin 2e (in red, third vertical row), and loricrin (in red, fourth vertical row) labelled by specific antibodies. For comparison the nuclei are stained by DNA dye (Hoechst). (Same magnification in all figures, bar 100µm).

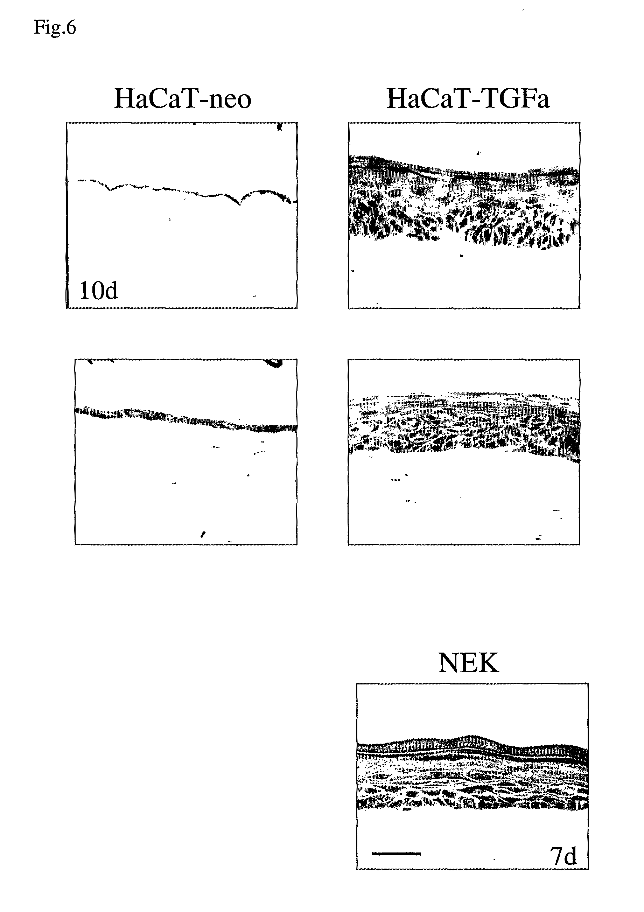

### Figure 6: Morphology (histologic H&E staining) of organotypic cocultures of transfected HaCaT cells

HaCaT cells transfected with the control pIRESneo2 vector (HaCaT-neo) or the pIRES neo2-TGFα vector (HaCaT-TGFα) were grown for 10 days as organotypic cocultures on collagen gel with 2x10⁵ resting fibroblasts/ml in the gel and grown in DMEM with 10% FCS and ascorbic acid. For comparison, a 7-day-old organotypic culture of normal skin keratinocytes (NEK) is shown to demonstrate the rather normal development of transfected HaCaT cells as compared to normal keratinocytes. (All figures are shown in the same magnification, bar 100µm).

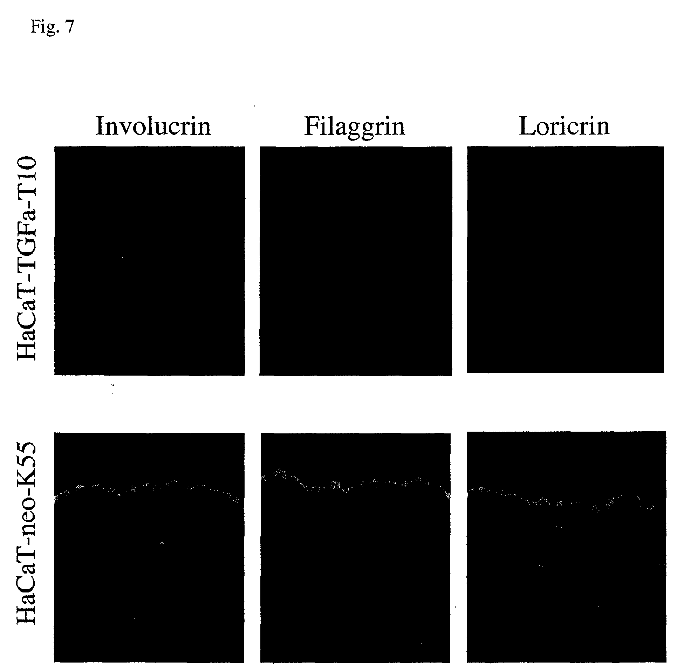

### Figure 7: Expression and localization of differentiation markers in organotypic cocultures with stably TGFα expressing HaCaT cells

By indirect immunofluorescence on cross sections the epidermal differentiation markers involucrin, filaggrin, and loricrin are labelled in HaCaT-TGFα epithelia (upper row) and in HaCaT-neo control epithelia (lower row). Nuclei were counterstained with bisbenzimide (blue). Epithelial border to the collagen gel is indicated by a dotted line. (Same magnification: bar 100µm).

The following examples further explain the present invention.

### Example 1: General methods

### A. Cultivation of HaCaT cells

Cryoconserved HaCaT cells (deposited under the Budapest Treaty with the "Deutsche Sammlung von Mikroorganismen und Zellkulturen GmbH (DSMZ), Mascheroder Weg 1 Braunschweig, Germany" on October 27, 1999: HaCaT-HSP70/Cat DSM ACC2417) were thawed in DMEM with 10% FCS and cultivated in the same medium with medium change every 2 to 3 days. 10⁴ cells/cm² were plated and passaged every 10 days with a split ratio of 1:10 as follows: the monolayer was rinsed with 0.05 % EDTA followed by 5-10 min. incubation with 0.05 % EDTA to cleave the cell junctions (desmosomes). For cell attachment the EDTA solution was replaced for 5-10 min. by the solution of trypsin:EDTA/1:1 and the cultures were incubated at 37°C. Detached cells were rinsed with the trypsin/EDTA solution and suspended in a three-fold amount of medium. Cell number was measured with an electronic counter (Casy1, Schärfe System, Reutlingen).

### B. Cloning of the transfection-vector

The human cDNA for TGFα including the ribosome and Poly-A-signal was cloned as a 0.9 kb TGFα-cDNA with the flanking EcoRI-site in a pBR327 vector carrying ampicillin resistance (ATCC No. 59950, Bethesda, Maryland, USA). Since the Poly-A-signal of the vector shall be used instead of the constitutive one of the TGFα cDNA, a 560 bp TGFα fragment only was used, positioned between an EcoRI and BalI cleavage site, and cloned into the expression vector. As expression vector pIRESneo2 vector with ampicillin resistance (Clonetech no. 6938-1; Heidelberg, Germany) was used so that the expression of the cloned cDNA was under control of the CMV promoter. Since this vector does not have a BalI restriction site in the multiple cloning site, the EcoRI/BalI-TGFα fragment was cloned in a shuttle vector via their restriction site EcoRI and BalI and cut via the restriction site EcoRI and NotI. Thereafter, the 570 bp EcoRI/NotI-TGFα fragment was cloned in the pIRESneo2 expression vector via the EcoRI and NotI restriction sites. The pIRES TGFα-plasmid was extracted in high purity and used in a concentration of 1µg/ml for transfection. The MT vector pIRESneo2 as control was used in the same concentration of 1µg/ml.

### C. Stable transfection

The stable transfection of HaCaT cells of passage 34 (standard HaCaT) was done following the protocol of the Gene-Jammer-Transfection Reagents (Stratagene, Germany, Heidelberg) with the proposed variants. In 60mm petri dishes (Falcon, Becton Dickinson, Germany, Heidelberg) 3x10⁵ and 4x10⁵ HaCaT cells, respectively, were plated, cultivated for 24 hours in DMEM with 10% FCS and further 24 hours kept in serum-free and antibiotic-free DMEM. Cultures with the two different plated cell numbers were transfected in 2.7 ml DMEM without antibiotics as follows:
1) 12 µl Gene-Jammer + 2 µl H₂0 (empty control)
2) 12 µl Gene-Jammer + 2 µg pIRESneo2 (control plasmid)
3) 12 µl Gene-Jammer + 2 µg pIRES-TGFα
4) 12 µl Gene-Jammer + 3 µg pIRESneo2
5) 12 µl Gene-Jammer + 2 µg pIRES-TGFα

Cultures were incubated for 5 hours and 37°C when 2.5 ml DMEM with 10% FCS were added per culture dish. The following day medium was changed with DMEM and 10% FCS. After two days, cells from each 60mm-petri dish were trypsinized, transferred into a 1OOmm-diamet dish and left for another 24 hours in DMEM with 10% FCS. Thereafter, dishes were incubated with the selection medium (Geneticin/Neomycin) with medium change every two days with decreasing concentrations of neomycin: 5 medium changes with DMEM/10% FCS and 600 µg/ml neomycin, further 5 times with 400 µg/ml neomycin and finally kept in DMEM with 10% FCS at 200 µg/ml neomycin. The surviving HaCaT colonies were ring-isolated and plated in 24 well plates, transferred after confluence in 12 well plates, followed by transfer in 6 well plates, 60mm dishes, 100 mm dishes and finally in 150mm dishes expanded before they were cryoconserved.

### D. Tests of the clones for transfection efficiency

The medium of confluent dishes which have been kept for 48 hours in 12 well plates were collected and examined by ELISA for TGFα production in comparison to the control transfectants (neo clones). Further tests of the TGFα production and release into medium by ELISA were done in 48 hours conditioned medium of confluent 100mm dishes. The positive and negative clones, respectively, were expanded on 150mm dishes and cryoconserved. Test of TGFα production was repeated after thawing the clones and before they are used in organotypic cocultures.

### E. Culture of fibroblasts

Primary fibroblasts were obtained as explant cultures from healthy human skin as described (Smola et al., J. Cell. Biol. 122 (1993), 417-429; Smola et al., Toxicol. In Vitro 8 (1994), 641-650; Stark et al., J. Invest. Dermatol. 112 (1999), 681-691) and cultivated in DMEM with 10% FCS. After plating at a density of 6x10³ cells/cm² cells are passaged at near confluence and medium was changed every 2 to 3 days. Fibroblasts until passage 9 were used for the production of organotypic cocultures. For passaging medium was discarded and the cells rinsed with 0.05% EDTA followed by the incubation in trypsin EDTA/1:1 for 5-10 min. After cell detachment, cell suspension was rinsed with a three-fold volume of DMEM and 10% FCS and cell count performed with an electronic counter (Caseyl, Scharfe-System, Germany, Reutlingen).

### F. Production of resting, postmitotic fibroblasts

In order to stop proliferation of fibroblasts and render them postmitotic, cells were γ-irradiated in suspension. For this trypsinized fibroblasts were centrifuged at 1000 rpm for 5 min. and the cell pellets dissolved in the desired medium type depending on the further protocol, so that the number was not exceeding 5x10⁵ cells/ml. This suspension was irradiated with 70 Gy in a Cs source. By this procedure, proliferation was permanently terminated, however, their phyiologic functions, in particular their interaction with keratinocytes was not essentially altered.

### G. Cryoconservation of resting, postmitotic fibroblasts

For the conservation of postmitotic fibroblasts irradiated cells were centrifuged at 1000 rpm for 5 min. and resuspended in 4°C freezing medium at a density of 1.5x10⁶ cells/ml. This cell suspension was transferred into freezing vials at 1 ml aliquots. The freezing vials were incubated in a precooled freezing box (filled with isopropanol) for 1 hour at 4°C and finally stored overnight at -80°C. Thereafter, the cells were stored in the gas phase of liquid nitrogen. Before final use the cells were tested concerning sterility and viability.

### H. Organotypic cocultures

For organotypic cocultures resting (irradiated) fibroblasts were embedded into collagen gels. The collagen gels consist of 80 volume % of a solution of 4 mg/ml collagen type I dissolved in 0.1 acidic acid. This solution was kept on ice and dissolved with 10 volume % ice-cold Hanks solution (with phenol red) and neutralized with 5 M NaOH. Irradiated fibroblasts were trypsinized, centrifuged at 1000 rpm for 5 min. and resuspended in FCS at a cell concentration, so that the final collagen gel contained 2x10⁵ cells/ml with FCS amounting to 10 volume % of the final collagen gel. This cell suspension in FCS was gently distributed in the collagen solution. The collagen solution containing the cells was plated into filter inserts (3 µm pore size, Falcon, Becton Dickinson) in special 6 well plates (deep-well plates, BioCoat, Becton Dickinson; see Figure 1). 2.5 ml of collagen solution was pipetted into each insert and care was taken that no air bubbles were included. The collagen gels were incubated for 1 hour at 37°C in the incubator leading to gelation of the material.

Sterile glass rings with an inner diameter of 18 mm (1.5mm wall size, 12mm height) were placed on the solidified collagen gels and gels were further incubated for 1 hour in the incubator at 37°C. Thereafter, the solution on top of the gel was pipetted and replaced by DMEM with 10% FCS both on top and beneath the collagen gels so that they were completely covered by medium. After 24 hour equilibration with complete culture medium the medium in the glass ring was replaced by 1 ml HaCaT cell suspension at a concentration of 1x10⁶ cells/ml. 48 hours later the medium on top of the plated HaCaT cells was discarded or pipetted and the glass ring eliminated. Starting with this time, the cells were cultured air-exposed (indicating day 0 of the organotypic coculture period). For the following culture period no medium was added to the upper surface of the plated HaCaT cells but medium was only changed in the lower culture compartment where cytokines or other medium additives were added to the cultures, e.g. DMEM with 10% FCS + 50 µg/ml ascorbic acid + 1ng/ml human EGF + 1ng/ml human TGFα and 1Ong/m human KGF or 100 ng/ml GM-CSF, respectively. Medium was changed every 2 to 3 days and care was taken that the upper miniscus did not reach the upper rim of the collagen gel so that the epithelia always remain air-exposed (see Figure 1).

### I. Preparation of the skin equivalents for histology and cryosectioning

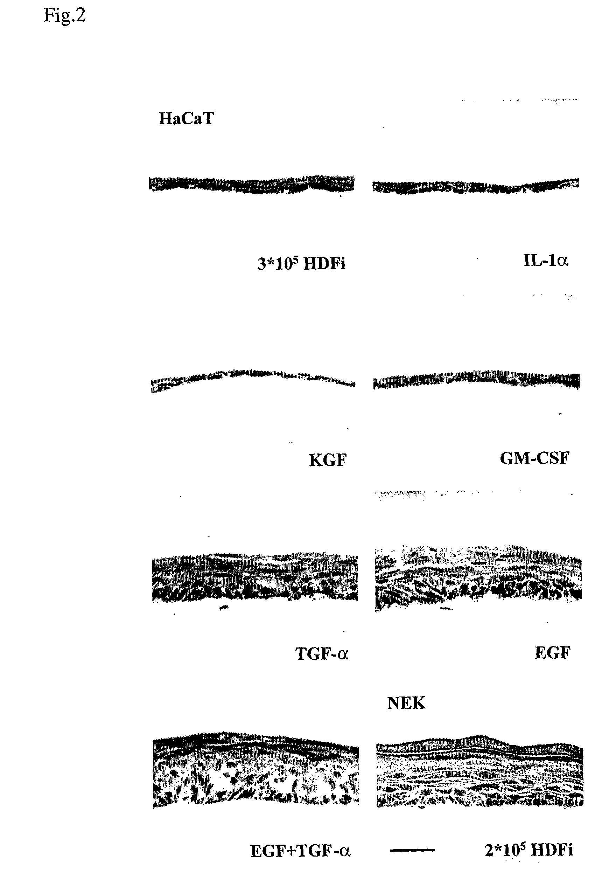

At indicated times, filter inserts are taken of the multiwell plates and cultures were cut with a scalpel together with the filter out of the inserts. For paraffin embedding and sectioning specimens were fixed for 24 hours in 3.7 formaldehyde in PBS, embedded in 0.3% agar to prevent dislodgement of the different parts and fixed for another 24 hours in formaldehyde. Specimens were then embedded in capsules (Medim, Germany, Gießen), dehydrated in increasing alcohol solutions and xylol and embedded in paraffin (standard histology procedures). 4 µm sections were cut from blocks, stained with hemalaun (15 min), eosin (5 min) and embedded in eukitt (Medim) (see Figures 2, 3, 6).

For cryosectioning organotypic cultures were placed on cork plates in tissue tec frozen in the gas phase of liquid nitrogen and stored at -80°C. 7 µm sections were cut on a cryotome, placed on coated slides and stained with the appropriate antibodies for immunofluorescence microscopy using specific antibodies against the indicated differentiation markers such as filaggrin, keratin 2e or loricrin (see Figures 5 and 7).

### J. Material used for the preparation of HaCaT organotypic cocultures

EDTA (Ethylendiamintetraacetate); Merck Darmstadt (0.05% in PBS with phenol red)
FCS (Fetal calf serum); Biochrom, Berlin
Hanks Salt solution with 8 mg/l phenol red; Biochrom, Berlin
Collagen Type I: (Rat tail tendon), own production; bovine skin collagen; IBFB, Leipzig
Trypsin (0.1% in PBS); Roche Diagnostics, Mannheim
Cell Culture Dishes No. 3003, 3025; Falcon BD, Heidelberg
Deep Well Plates (6-well, Biocoat, No. 355467) Falcon, BD
Filter Inserts (3 µm Filter, No. 353092) Falcon, BD
NaOH (5 M); Merck
DMEM; Biochrom
hEGF *(human epidermal growth factor),* 1 ng/ml; Sigma, Deisenhofen
hTGF-alpha *(human transforming growth factor alpha),* 1 ng/ml; R&D Systems, Wiesbaden
hKGF *(human keratlnocyte growth factor),* 10 ng/ml; BTS, St. Leon
hGM-CSF *(human granulocyte-macrophage colony stimulating factor),* 100 ng/ml; BTS, St. Leon
Asc (Ascorbic Acid), 50 µg/ml; Sigma
Freezing medium: DMEM, 10% Glycerol, 20% FCS
sterile beakers; magnetic stirrers; icebox; incubator.

### K. Material used for the production of stable HaCaT-TGFα-clones

TGF-α cDNA; ATCC Nr. 59950, Bethesda, Maryland, USA Restriction enzymes; Roche Diagnostics, Mannheim pIRESneo2 Vektor; Clontech Nr. 6938-1, Heidelberg Gene-Jammer-Transfection-Reagent; Stratagene, Heidelberg human TGF-α ELISA; Oncogene (Merck, Darmstadt)

### Example 2: Production of stable HaCaT/TGFα cell clones and their use in organotypic cocultures with fibroblasts

Studies of the present inventors clearly demonstrated that neither in monolayer nor in three-dimensional organotypic cultures neither the keratinocyte growth factor (KGF) nor the granulocyte macrophage stimulating factor GM-CSF were able to stimulate and structure the organization of HaCaT cells (Figure 2). Furthermore, it could be observed that the receptors of these two growth factors (KGF and GM-CSF) were produced to minor extents only at the RNA level in HaCaT cells but the protein at the cell membrane in contrast to normal keratinocytes was not detectable in proliferating low density cell cultures. Finally, it could be elucidated another deficit of HaCaT cells in the epithelial-mesenchymal interaction and the growth regulation based on this interaction which had been documented to be essential for normal keratinocyte function. The production of 11-1 released in large amounts in normal keratinocytes following wounding or stress (UV) was drastically reduced in HaCaT cells. RNA expression was still detectable, although at a very low level, but the secreted protein was nearly completely missing. And last but not least, the RNA expression of the autocrine keratinocyte transforming growth factor α (TGFα) was drastically reduced in HaCaT cells, whereas the secreted protein could not be detected in the ELISA test.

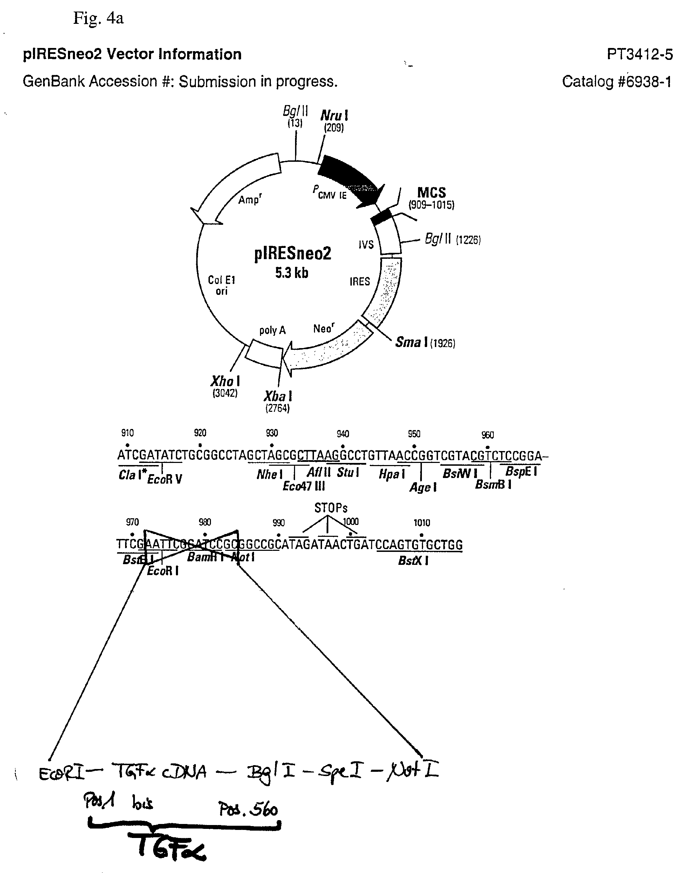

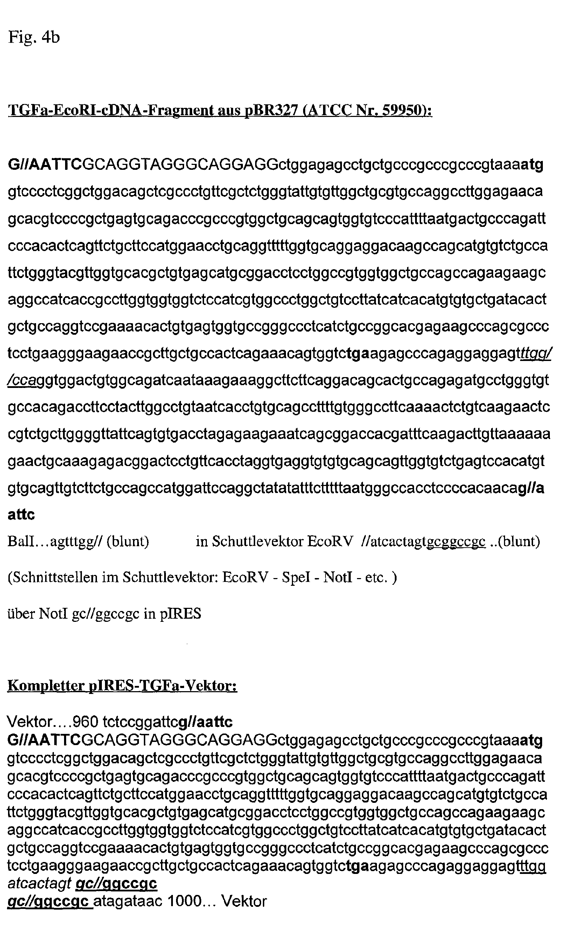

These deficiencies, e.g., the deficiency in structural tissue formation and differentiation of HaCaT cells in organotypic coculture with fibroblasts could be normalised by the induction and increase of the missing factors and by the enhancement of expression of the receptors of KGF and GM-CSF by addition of TGFα and EGF, respectively, both binding to the same EGF receptor. After this induction at the RNA and protein level HaCaT cells became sensitive for KGF and GM-CSF resulting in an increased formation of a new epithelium with drastically reduced apoptosis of the proliferating cells and formation of a regular structured squamous epithelium. Importantly, a stratum granulosum appeared and the stratum corneum was significantly improved, both indications of a regular differentiation and an improved barrier function of the in vitro skin equivalent. Comparable stimulating effects in HaCaT cell proliferation and differentiation have been achieved after pretreatment of cells with TGFα and addition of Il-1 which induced KGF and GM-CSF in the cocultured fibroblasts (Figure 3). The compensation of the defects in HaCaT cells in the synthesis of the autocrine growth factor TGFα has been achieved much more efficiently and with a constant secretion of these growth factors by stable transfection of a TGFα cDNA under the control of a constitutive active promoter (CMV) in a commercially available plasmid (Figures 4a and b). By a commercially available transfection kit (Gene-Jammer, Stratagene, Germany, Heidelberg) multiple transfectant clones exhibiting resistance towards the selection gene neomycin could be isolated. Seven of those showed measurable TGFα secretion, although at different intensity. Two of these HaCaT/TGFα cell clones produced reasonable amounts of TGFα comparable to that measured in normal keratinocytes. (For detailed description of transfection, selection and characterisation see Example 1).

These HaCaT/TGFα transfectants exhibited in organotypic cocultures with normal skin fibroblasts a rapid and regular epithelial reconstruction with normal differentiation (Figure 6) as well as expression of the receptors for KGF and GM-CSF. By further addition of GM-CSF differentiation of the HaCaT epithelia is further improved resulting in a regular keratinization and formation of a normal stratum corneum comparable to that of normal keratinocytes and responsible for normal barrier function (Figure 6).

Since this normalisation of differentiation occurs also in coculture with resting (postmitotic, i.e. irradiated) fibroblasts incorporated in collagen gels, a further standardisation of this model system has been achieved by keeping the fibroblast number constant throughout the whole experimental period. Untreated, i.e. proliferating fibroblasts exhibit a variable rate of proliferation and apoptosis in the collagen gel depending on culture conditions, culture periods and cellular origin resulting in constant variations of their effect on proliferation and differentiation of HaCaT cells as well as normal keratinocytes. Using a defined culture medium free of any addition of serum or organ extracts which equally well supported normal differentiation of keratinocytes in organotypic cultures (Stark et al., 1999), a further variable component in producing these organotypic skin equivalents has been standardised leading to improved reproducibility.

Thus, following stable transfection and expression of TGFα in immortalised HaCaT cells and the consequence of induction of KGF and GM-CSF receptors, an in vitro skin equivalent has been produced. Its differentiation capacity is further improved by the addition of Il-1 or the (fibroblast-induced) growth factors KGF and GM-CSF. Therefore, with these genetically modified HaCaT cells an in vitro epidermis could be created with properties very comparable to that established of normal keratinocytes (Figure 6). The quality of differentiation in the HaCaT epithelium was obvious by morphology, the regular localisation of the typical differentiation products Keratin 1/10, involucrin, filaggrin and loricrin (Figure 7) (for details see Example 1).

## Claims

1. An immortalized mammalian skin keratinocyte line stably transfected with a vector capable of expressing a nucleic acid sequence encoding at least one growth factor and/or activated EGF receptor.

2. The immortalized mammalian skin keratinocyte line of claim 1, wherein the growth factor is an EGF receptor binding growth factor.

3. The immortalized mammalian skin keratinocyte line of claim 1 or 2, wherein the growth factor is TGFα, EGF, HB-EGF or amphiregulin.

4. The immortalized mammalian skin keratinocyte line of claim 1, 2 or 3, which is HaCaT.

5. The immortalized mammalian skin keratinocyte line of claim any of claims 1 to 4, wherein the nucleic acid sequence encoding the growth factor and/or activated EGF receptor is operatively linked to a CMV promoter.

6. The immortalized mammalian skin keratinocyte line of claim 4, deposited with the DSMZ under DSM ACC2545 on June 5, 2002.

7. The immortalized mammalian skin keratinocyte line of any one of claims 1 to 5 which further contains a reporter gene operatively linked to a promoter inducible by environmental hazards.

8. The immortalized mammalian skin keratinocyte line of any of claims 1 to 7 stably transfected with one or more vectors expressing two or more growth factors and/or activated EGF receptors.

9. A mammalian skin equivalent comprising a discrete epidermal layer of the immortalized mammalian skin keratinocyte line of any one of claims 1 to 8 on top of a gel matrix containing fibroblasts.

10. A mammalian skin equivalent comprising a discrete epidermal layer composed of a mixture of (a) the immortalized mammalian skin keratinocyte line of any one of claims 1 to 8 and (b) keratinocyte cells permanently transfected with a reporter gene operatively linked to a promoter inducible by environmental hazards on top of a gel matrix containing fibroblasts.

11. A method for forming a skin equivalent comprising
(a) preparing a gel matrix with or without embedded fibroblasts and applying a suitable medium on the surface of said matrix;
(b) replacing the medium with a suspension comprising cells of the immortalized mammalian skin keratinocyte line of any one of claims 1 to 8; and
(c) cultivating the cells of (a) and (b).

12. The mammalian skin equivalent of claim 9 or 10 or the method of claim 11, wherein the gel matrix is a collagen matrix.

13. The method of claim 11 or 12, wherein the suspension further comprises keratinocyte cells permanently transfected with a reporter gene operatively linked to promoter inducible by environmental hazards.

14. The method of any one of claims 11 to 13 further comprising the addition of mammalian (a) Il-1 or (b) KGF and/or GM-CSF in step (c).

15. The method of any one of claims 11 to 14, wherein the fibroblasts are resting postmitotic fibroblasts.

16. An in vitro assay for identifying a candidate agent or environmental hazard having an affect on skin comprising
(a) providing the mammalian skin equivalent of claim 9, 10 or 12 or the mammalian skin equivalent prepared according to the method of any one of claims 11 to 15;
(b) contacting said skin equivalent with a candidate agent or applying a candidate environmental hazard to said skin equivalent; and
(c) determining whether said candidate agent or environmental hazard has an effect on said skin equivalent or whether expression of the reporter gene occurs, wherein expression of the reporter gene is indicative of cell toxicity of the candidate environmental hazard.
